# EUROPEAN PATENT SPECIFICATION

(11) **EP 0 948 318 B1**
(45) Date of publication and mention of the grant of the patent: **24.03.2004**
(21) Application number: 97944780.2
(22) Date of filing: 14.08.1997
(51) Int. Cl.: A61K 9/16

(54) **GRANULATES COMPRISING A WATER SOLUBLE COMPOUND AND CELLULOSE**
EINE WASSERLÖSLICHE VERBINDUNG UND CELLULOSE ENHALTENDE GRANULATE
GRANULES COMPRENANT UN COMPOSE SOLUBLE DANS L'EAU ET DE LA CELLULOSE

(30) Priority: 14.08.1996 EP 96202289
(43) Date of publication of application: 13.10.1999
(73) Proprietor: YAMANOUCHI EUROPE B.V., 2350 AC Leiderdorp (NL)
(72) Inventor: VAN KOUTRIK, Robertus C., Yamanouchi Europe B.V., 2350 AC Leiderdorp (NL); SIJBRANDS, Gerrit Jan, Yamanouchi Europe B.V., 2350 AC Leiderdorp (NL)
(86) International application number: PCT/EP1997/004570
(87) International publication number: WO 1998/006382

(56) References cited:
- EP-A- 0 330 284
- WO-A-93/12768
- US-A- 5 049 394
- BANSAL P ET AL: "EFFECT OF COMPRESSION ON THE RELEASE PROPERTIES OF POLYMER COATED NIACIN GRANULES" JOURNAL OF CONTROLLED RELEASE, vol. 27, no. 2, 1 November 1993, pages 157-163, XP000400404

## Description

The present invention relates to compositions containing a granulate containing a water soluble compound.

### BACKGROUND OF THE INVENTION

Generally, if a quick onset of action of a drug from a solid dosage-form, such as a tablet, is aimed at, the composition and the manufacturing method of such tablet will be carefully selected as to enable a fast disintegration of the tablet and a dissolution of the drug, when placed in an aqueous environment. EP-B-0330284 discloses a process for the preparation of a pharmaceutical granulate by granulating a drug, having a solubility in water of less than 10 wt%, and 20-100 wt% of microcrystalline cellulose with water without using a substantial amount of a wet granulation binding agent. The amount of water to be used ranges between about 60 wt% and 135 wt%, the percentages based on the weight of the active ingredient. From a granulate, obtained in this way, fast-disintegrating tablets can be prepared.

However, the incorporation of active ingredients which have a solubility in water of more than 10 wt% into tablet compositions may present problems as to the disintegration thereof and consequently the dissolution of the active ingredient. Z.T. Chowhan et al. (1982) Drug Developm. Industr. Pharm. 8 (2), page 145-168 mentioned that tablets containing a high percentage (66 wt%) of a hygroscopic and water soluble drug (naproxen sodium) did not disintegrate when a dissolution test was performed. The drug mainly dissolved at the effective surface area of the tablet, which was exposed to the dissolution medium. Consequently, large tablet-to-tablet variability of such tablets in the dissolution test was observed. The addition of disintegrating agents (sodium starch glycolate and sodium carboxymethyl cellulose in addition to 10% of starch) neither improved dissolution variability nor increased the rate of drug dissolution. The results further suggested that the formulation in which a major portion of the excipients was not wet granulated together with the drug, resulted in a higher tablet-to-tablet dissolution variability. In view of these results it appeared to be very difficult to develop a tablet formulation containing a water soluble active ingredient which will give reliable and reproducible effects when administered to humans or animals.

A well-known dosage-form for water soluble compounds is an effervescent tablet. On placing such tablet in water it readily disintegrates and dissolution should be achieved within a few minutes. However, it is not convenient to administer these tablets without dissolving the tablets in water first, due to the simultaneous release of carbon dioxide. Another disadvantage of an effervescent tablet and an important reason for its somewhat limited utilisation, is related to the difficulty of producing a chemically stable product, due to the moisture-sensitivity of the effervescent couple.

Since many active compounds, such as drugs, are water soluble, the problem to be solved by the present invention was to provide a formulation or manufacturing method, which would be generally applicable to all kinds of water soluble active ingredients, for fast-disintegrating and fast-dissolving compositions.

### SUMMARY OF THE INVENTION

A composition containing a granulate, consisting of a water soluble active ingredient, 0-100 wt% of a cellulose product, such as microcrystalline cellulose, and 0-0.5 wt% of a wet granulation binding agent, all percentages based on the weight of the active ingredient, the granulate, prepared according to a process which includes moistening the component(s) to be granulated with an essentially aqueous liquid without using a substantial amount of a wet granulation binding agent and consecutively comminuting the wet mass, drying the granules and sieving these, a cellulose product such as microcrystalline cellulose, and a disintegrant, is provided.

### DETAILED DESCRIPTION OF THE INVENTION

A water soluble active ingredient according to the present invention is a substance which is soluble in water at room temperature in a ratio of 1: ≤ 10. There are numerous examples of such compounds, which can be found among all kinds of physiologically and cosmetically active substances. The most important active substances are vitamins, nutritional agents and drugs. The vitamins include e.g. ascorbic acid, pyridoxine hydrochloride and thiamine hydrochloride. Nutritional agents are various amino acids. Water soluble drugs can be found in many different classes of therapeutical compounds: mucolytics, such as N-acetyl-cysteine and S-carboxymethyl-cysteine; antimicrobial agents, such as neomycine sulphate and the water soluble salts of beta-lactam antibiotics; iron salts, such as ferrous gluconate and ferric ammonium citrate; tuberculostatics and tuberculocides, such as ethambutol hydrochloride and isoniazide; beta-adrenoceptor blocking agents, such as metoprolol tartrate and sotalol hydrochloride; certain biphosphonic acids and the salts thereof; antimalarials, such as various quinine salts etc. Preferred water soluble compounds are the salts of the β-lactam antibiotics:pheneticillin potassium, the sodium and potassium salt of phenoxymethylpenicillin and the sodium salt of flucloxacillin.

The granulate according to the invention may essentially consist of the water soluble active ingredient or may contain the same ingredient in admixture with commonly used excipients, which are cellulose products, such as microcrystalline cellulose, microfine cellulose or a mixture thereof, in a concentration of up to and including 100 wt%, the percentage based on the weight of the active ingredient. Advantageously the granulate contains at least 50 wt% of the water soluble compound. In particular when high doses of such compound have to be incorporated in a swallowable oral composition, the use of large amounts of excipients should be avoided as much as possible. Preferred concentrations of the water soluble active ingredient in the granulate are at least 75 wt%, but more preferably at least 85 wt%.

The granulate is prepared at room temperature by a wet granulation technique, using an essentially aqueous liquid as the granulating liquid, however without using substantial amounts of wet granulation binding agents. The granulating liquid may contain up to 10 wt% of ethanol. In the said aqueous liquid up to and including 0.5 wt%, preferably 0-0.1 wt%, of a wet granulation binding agent may be dissolved, the percentages based on the weight of the active ingredient. Suitable wet granulation binding agents include water soluble celluloses, such as hydroxypropyl celluloses and sodium carboxymethyl cellulose, starches (soluble, pregelatinised), polyvinylpyrrolidone, although naturally occurring binders, such as acacia gum, corn starch, sugars and polyhydroxy compounds, such as mannitol, can also be used.

The amount of granulating liquid to be used is dependent on the active ingredient, the ratio between the active ingredient and the cellulose product and the composition of the granulating liquid and may range from 1 to 40 wt% and preferably from 2.3 to 20 wt%, the percentages based on the weight of the active ingredient. The granulate may be prepared by gradually adding the essentially aqueous solution, optionally containing up to and including 0.5 wt% of the wet granulation binding agent, to the component(s) to be granulated and mixing the mass during 15 to 25 minutes, preferably 20 minutes, and subsequently screening the wet mass thus obtained through an at least 2.0 mm sieve. Alternatively, the greater part of the water soluble compound, optionally mixed with the cellulose product, may be granulated with an aqueous solution, containing the remaining part of the same compound. After drying the granules in a fluidised bed dryer at an inlet air temperature of between 30 and 60°C, preferably 45°C, these are screened again, but now through a sieve having pores of at least 0.71 mm, but preferably between 1.00 and 1.50 mm. Alternatively, the wet mass can be comminuted, in e.g. a hammer mill. The particle size distribution of the granulate should meet special requirements for incorporation into the fast-disintegrating or quickly dispersible compositions according to the present invention. A suitable distribution is e.g.:

| | |
|---|---|
| ≥ 1.400 mm | 0.3% |
| 1.000-1.400 mm | 28.2% |
| 0.710-1.000 mm | 23.8% |
| 0.500-0.710 mm | 20.2% |
| 0.355-0.500 mm | 12.7% |
| 0.250-0.355 mm | 7.6% |
| 0.180-0.250 mm | 3.6% |
| 0.125-0.180 mm | 2.3% |
| 0.090-0.125 mm | 0.8% |

Suitable apparatus for production of the granulate include a planetary mixer, but also a fluid bed granulator, a high shear mixer or a high speed mixer. It has been observed that the optimal mixing time depends on the apparatus used, the mixing speed and the particle size of the powders to be granulated. In case a high speed mixer is used a considerable reduction of mixing times could be achieved.

The granulates obtained according to the present invention show satisfactory properties, such as a good flow and a Hausner ratio. Dissolution studies revealed a fast dissolution of the active ingredient. It is another advantage that an organic solvent, with all safety and environmental hazards, as a granulating liquid can be avoided. Furthermore, it has been observed that the taste of the active ingredient, if deemed necessary, can be relatively easily improved by means of the addition of sweetening agents and flavours to the granules according to the present invention. Panel tests, performed on volunteers, have shown that taste-masking of a water soluble β-lactam antibiotic, such as phenoxymethylpenicillin potassium, in granular form is not more of a problem than the taste-masking of granulates containing a slightly soluble form of the same antibiotic (phenoxymethylpenicillin). It is advantageous that a granulate, containing a water soluble drug for the greater part, can be obtained by a wet granulation technique using an essentially aqueous liquid as the granulation liquid especially since normally a liquid in which the powders to be granulated are only slightly soluble would be selected as the granulating liquid. Despite the fact that the solubility of a compound in the granulating liquid is high and the powder which is in contact with the granulating liquid will dissolve in it, it has been possible to evenly distribute the liquid throughout the powder. Overwetting of the powders to be granulated could be prevented as well as the formation of very hard string-shaped granules after wet screening and drying, which granules are not suitable for compression into tablets without further processing. Furthermore, it is an advantage that the wet screening process proceeds very smoothly because the pores of the screen will not gradually silt up.

For obtaining the quickly dispersible compositions according to the present invention, which can be orally administered, the water soluble active ingredient in a granular form is blended with a cellulose product, which is microcrystalline cellulose, microfine cellulose or a mixture thereof, one or more disintegrants, and optionally sweetening agents, flavours and aromas, lubricants, anti-adhesives, flow-promoters etc.

The total percentage of active ingredient and cellulose product may range from about 80 to about 85 wt%, the percentage based on the final composition. The ratio of active ingredient and cellulose product may vary from 1:0.5 to 1:1, the preferred ratio being 1:0.9 (total of intra- and extragranular amounts).

The cellulose product can have a mean particle size ranging from 50 to 250 µm, but preferably a product having a mean particle size of 100 µm, such as the microcrystalline cellulose type Avicel® PH 102, is used. If a reduction of the height of the compressed composition is aimed at, the cellulose product can be partially substituted by a calcium phosphate, which is commercially available under the trademark Emcompress®. However, an increase of the disintegration time of such compressed compositions has been observed.

The disintegration time of the solid oral compositions according to the invention, such as capsules and tablets, is determined by means of a disintegration time apparatus, (ERWEKA) operated without using discs, e.g. according to the European or British Pharmacopoeia, however with a further modification of the movement (22 mm instead of 55 mm) simulating a user's situation. The maximal disintegration time of the compositions according to the invention is 2 minutes, but preferably less than one minute. It is also possible to determine a dispersion time, especially of sachet-formulations, in a beaker. Furthermore, dispersible tablets should also meet the requirements of the European or British Pharmacopoeia for such tablets.

Useful disintegrants appear to be among others super disintegrants and include modified starches, such as sodium starch glycolate, cross-carmellose, sodium carboxymethyl cellulose, cross-linked polyvinylpyrrolidone, polacrilin potassium (Amberlite® IRP 88), and low-substituted hydroxypropyl cellulose. The said disintegrants, alone or in combination, can be advantageously used in the compositions in order to let these quickly disperse when placed in an aqueous environment.

The disintegrant or mixture of disintegrants is generally used in a concentration of between 6 and 15 wt%, the percentage based on the composition. However, preferably 7-11 wt% and most preferably 8.5-9.5 wt% is used.

The granular form of the water soluble active ingredient, to be used in compositions, can be obtained according to the process, as described above. However, in case of very bitter compounds it may be useful to add a filmcoating to the granules, containing the water soluble active ingredient. This can be done after preparing the granulate as described above, but it is also possible during the granulating process. Film-forming polymers are e.g. cellulose derivatives and acrylic acid based polymers. Due to the solubility characteristics of the last mentioned commercially available film-forming agents usually no aqueous liquid can be used as the solvent. Advantageous results have been obtained with granulates, prepared by the wet granulation technique, using an alcoholic solution of film-forming polyacrylates, such as Eudragit® E100. On applying an aqueous dispersion of film-forming polyacrylates, such as Eudragit® E30D and RL30D, the granulation and filmcoating process can be combined. The concentration of the film-forming agent in the composition may be up to 5 wt%. Granulates can be prepared with aqueous solutions containing up to 5 wt%, but preferably up to 0.5 wt%, the percentage based on the composition of the film-forming cellulose derivatives such as hydroxyethyl cellulose, hydroxypropyl cellulose, hydroxypropylmethyl cellulose and sodium carboxymethyl cellulose. The active ingredient in granular form, obtained according to any of the methods described above, should have the same characteristics with respect to particle size and flow-properties, as the granulates. prepared by the process, using an aqueous solution containing no substantial amount of wet granulation binding agent.

The compositions according to the present invention are very versatile in that they can be used for the preparation of quickly dispersible and/or disintegrating capsules, tablets. sachet-presentations etc. The bioavailability of the active ingredient from the composition, when dispersed in water prior to administration or swallowed as such, is similar. As already mentioned the taste of the active ingredients can be relatively easily masked by a careful selection of sweetening agents and flavours. Sugars or polyhydroxy compounds are no longer required.

The following examples further illustrate the invention.

### EXAMPLES

### Examples 1-7

400-800 g of the water soluble compound, as mentioned in table 1, were granulated at room temperature with water in the amount, as specified in table 1, during 20 minutes in a planetary mixer (HOBART). Thereafter the wet mass was screened through an at least 2.0 mm sieve (2.0-4.0 mm). The granulate was subsequently dried in a fluid bed dryer (RETSCH) during 30-45 minutes at an inlet air temperature of 45°C. Then the dry product was screened in an oscillating granulator (FREWITT or ERWEKA), equipped with a 1.0 or 1.25 mm sieve.

### Examples 8-10

800 g of the water soluble compound, as mentioned in table 2, were granulated at room temperature with an aqueous granulation liquid containing 10% of ethanol in the amount, as specified in table 2, according to the method as described in examples 1-7.

### Examples 11-13

800 g of a water soluble compound, as mentioned in table 3, and 133.3 g of microcrystalline cellulose were granulated with water in the amount, as specified in table 3, during the period, shown in the same table, in a planetary mixer (HOBART). After comminuting the wet mass the granulate was subsequently dried in a fluid bed dryer (RETSCH) during 30 minutes at an inlet air temperature of 45°C. Finally the dry product was screened in an oscillating granulator (FREWITT or ERWEKA), equipped with a 1.25 mm sieve.

### Examples 14-19

Phenoxymethylpenicillin potassium optionally mixed with microcrystalline cellulose (see table 4) was granulated with water (see table 4) at room temperature during 20 minutes in a planetary mixer (HOBART). Thereafter the wet mass was screened through a 2.0 mm sieve. The granulate was subsequently dried in a fluid bed dryer (RETSCH) during 35 minutes at an inlet air temperature of 45°C. Then the dry product was screened in an oscillating granulator (FREWITT), equipped with a 1.25 mm sieve.

### Examples 20-25

800 g of ferrous gluconate, thiamine hydrochloride or ascorbic acid, mixed with an amount of microcrystalline cellulose, as shown in tables 5, 6 and 7 respectively, were granulated with an amount of water, as specified in the same tables, according to the method described in examples 11-13.

### Example 26

The granulates obtained according to examples 1-25 were blended with the excipients according to the formula herebelow in a mixer (TURBULA) and compressed into tablets using a flat bevel edge, round 14 mm mould. The tablets had a mean weight of about 900 mg and had the properties, as shown in tables 1-7:

| | |
|---|---|
| water soluble compound | 400 mg |
| microcrystalline cellulose (total of intragranular and extragranular amount) | 365 mg |
| cross-linked polyvinylpyrrolidone | 73 mg |
| silicone dioxide | 1 mg |
| magnesium stearate | 5 mg |
| flavours | q.s. |

**Table 1**

| Example | Water soluble compound | Amount of water (wt%)* | Tablet disintegration time (s) | Tablet Hardness (kP) | Tablet Friability (%) |
|---|---|---|---|---|---|
| 1 | N-acetylcysteine | 15.0 | 32 | 12.1 | 0.06 |
| | | | 53 | 14.9 | 0.10 |
| 2 | Ferrous gluconate | 12.5 | 17 | 6.6 | 0.90 |
| | | 8.1 | 16 | 7.7 | - |
| 3 | Thiamine | 15.0 | 20 | 6.8 | - |
| | hydrochloride | 10.0 | 20 | 6.2 | 0.38 |
| 4 | Pyridoxine | 20.0 | 20 | 6.8 | |
| | hydrochloride | 12.5 | 21 | 7.1 | 0.20 |
| 5 | Isoniazide | 20.0 | 22 | 7.6 | 0.90 |
| | | 12.5 | 19 | 4.9 | - |
| 6 | Metoprolot tartrate | 5.7 | 35 | 6.4 | 0.30 |
| | | 3.1 | 40 | 5.2 | - |
| 7 | Ascorbic acid | 15.0 | 24 | 9.7 | 0.24 |
| | | | 41 | 11.9 | 0.23 |
| | | 7.50 | 20 | 8.7 | - |
| | | 6.25 | 16 | 6.5 | 0.82 |

| | | | | | |
|---|---|---|---|---|---|
| * based on the weight of the active ingredient | | | | | |

**Table 2**

| Example | Water soluble compound | Amount of granulation liquid (wt%)* | Tablet disintegration time (s) | Tablet Hardness (kP) | Tablet Friability (%) |
|---|---|---|---|---|---|
| 8 | Ferrous gluconate | 12.5 | 16 | 7.4 | 0.13 |
| 9 | Thiamine hydrochloride | 15.0 | 19 | 8.3 | 0.15 |
| 10 | Ascorbic acid | 7.5 | 21 | 8.6 | 0.67 |

| | | | | | |
|---|---|---|---|---|---|
| * based on the weight of the active ingredient | | | | | |

**Table 3**

| Example | Water soluble compound | Amount of water | Granulation time (min.) | Tablet disintegration time (s) | Tablet Hardness (kP) | Tablet Friability (%) |
|---|---|---|---|---|---|---|
| 11 | Neomycine sulphate | 125 ml | 20 | 13 | 5.8 | 0.47 |
| 12 | N-acetyl-cysteine | 150 ml | 30 | 25 | 9.7 | 0.13 |
| | | | | 46 | 12.8 | 0.12 |
| 13 | Ascorbic acid | 150 ml | 30 | 18 | 7.5 | 0.22 |
| | | | | 32 | 9.1 | 0.23 |

**Table 4**

| Example | Amount of microcrystalline cellulose (wt%)* | Amount of water (wt%)* | Tablet disintegration time (s) | Tablet Hardness (kP) | Tablet Friability (%) |
|---|---|---|---|---|---|
| 14 | 12.5 | 18.7 | 35 | 7.1 | 0.1 |
| 15 | 43.75 | 26.8 | 63 | 5.7 | 0.33 |
| 16 | 0 | 12.5 | 21 | 5.5 | 0.66 |
| 17 | 0 | 13.7 | 17 | 4.8 | 1.09 |
| 18 | 0 | 15 | 18 | 5.1 | 0.74 |
| 19 | 0 | 16.2 | 17 | 5.3 | 0.62 |

| | | | | | |
|---|---|---|---|---|---|
| * based on the weight of the active ingredient | | | | | |

**Table 5**

| Example | Amount of microcrystalline cellulose (wt%)* | Amount of water (wt%)* | Tablet disintegration time (s) | Tablet Hardness (kP) | Tablet Friability (%) |
|---|---|---|---|---|---|
| 20 | 33.3 | 26.6 | 43 | 8.5 | 0.10 |
| 21 | 100.0 | 40.0 | 53 | 7.7 | 0.17 |

| | | | | | |
|---|---|---|---|---|---|
| * based on the weight of the active ingredient | | | | | |

**Table 6**

| Example | Amount of microcrystalline cellulose (wt%)* | Amount of water (wt%)* | Tablet disintegration time (s) | Tablet hardness (kP) | Tablet Friability (%) |
|---|---|---|---|---|---|
| 22 | 33.3 | 26.6 | 36 | 8.2 | 0.02 |
| 23 | 100.0 | 40.0 | 38 | 7.7 | 0.33 |

| | | | | | |
|---|---|---|---|---|---|
| * based on the weight of the active ingredient | | | | | |

**Table 7**

| Example | Amount of microcrystalline cellulose (wt%)* | Amount of water (wt%)* | Tablet disintegration time(s) | Tablet Hardness (kP) | Tablet Friability (%) |
|---|---|---|---|---|---|
| 24 | 33.3 | 22.5 | 23 | 8.5 | 0.01 |
| 25 | 100.0 | 40.0 | 48 | 11.8 | 0.51 |

| | | | | | |
|---|---|---|---|---|---|
| * based on the weight of the active ingredient | | | | | |

### Example 27

A granulate, containing ascorbic acid as the active ingredient, was prepared according to the method described in examples 1-7. From this granulate tablets were made having the composition, as shown in example 26, except that microcrystalline cellulose was substituted by microfine cellulose (Eicema G250®). The tablets thus obtained disintegrated in water within 31 seconds, had a hardness of 6.3 kP and a friability of 0.80%.

### Examples 28-32

A granulate, containing ascorbic acid as the active ingredient, was prepared according to the method described in examples 1-7. From this granulate tablets were made having the composition, as shown in example 26, except that cross-linked polyvinylpyrrolidone was substituted by another disintegrant in various concentrations. The total amount of microcrystalline cellulose and the disintegrant together was the same as in example 26. The properties of the tablets thus obtained are shown in table 8.

**Table 8**

| Example | Disintegrant | Amount of disintegrant (wt%)* | Tablet disintegration time (s) | Tablet Hardness (kP) | Tablet Friability (%) |
|---|---|---|---|---|---|
| 28 | crosscarmellose sodium | 6 | 30 | 7.6 | 0.57 |
| | | 8 | 35 | 7.9 | 0.72 |
| | | 15 | 66 | 6.7 | 1.25 |
| 29 | low-substituted | 6 | 22 | 7.6 | 0.57 |
| | hydroxypropyl | 8 | 31 | 7.9 | 0.54 |
| | cellulose | 15 | 48 | 7.5 | 0.74 |
| 30 | ion-exchange resin | 6 | 20 | 8.8 | 0.56 |
| | (Amberlite® IRP-88) | 8 | 20 | 8.4 | 0.51 |
| | | 15 | 31 | 7.5 | 0.99 |
| 31 | sodium carboxymethyl | 6 | 36 | 8.0 | 0.59 |
| | cellulose | 8 | 37 | 8.2 | 0.54 |
| | | 15 | 52 | 4.9 | 1.67 |
| 32 | sodium starch glycolate | 6 | 26 | 7.9 | 0.45 |
| | | 8 | 37 | 7.8 | 0.80 |
| | | 15 | 104 | 9.6 | 0.75 |

| | | | | | |
|---|---|---|---|---|---|
| * based on the weight of the composition | | | | | |

### Example 33

200 g of phenoxymethylpenicillin potassium were granulated with 30 ml of a solution containing 5% of soluble starch (Paselli® SA-2) in a planetary mixer during 20 minutes. After screening the wet mass through a 2.0 mm sieve and drying the product so obtained during 35 minutes in a fluidised bed dryer at an inlet air temperature of 45 °C, the granulating procedure was repeated. After drying the product obtained in the second granulating procedure, the granulate was screened in an oscillating granulator equipped with a 1.0 mm sieve.

### Example 34

4000 g of phenoxymethylpenicillin potassium were granulated with 550 ml of water in a high speed mixer operated at 200 rpm during periods between 55 and 120 seconds. Thereafter, the wet mass was screened through a 2.0 mm sieve. The granulate was dried in a fluid bed dryer at an inlet air temperature of 45°C during 35 minutes. The dry product was screened through a 1.0 mm sieve.

## Claims

1. Composition, containing a granulate, consisting of an active ingredient being soluble in water at room temperature in a ratio of 1:≤10, 0-100 wt% of a cellulose product, which is microcrystalline cellulose, microfine cellulose or a mixture thereof, and 0-0.5 wt% of a wet granulation binding agent, the granulate being prepared according to a process, comprising the steps of:
a. moistening component(s) making up the granulate with 1-40, preferably 2.5-20 wt%, of an aqueous solution optionally containing the wet granulation binding agent;
b. comminuting the wet mass through an at least 2 mm sieve;
c. drying the granulate obtained in step b;
d. screening the dried granulate through an at least 0.71 mm sieve,
a cellulose product, which is microcrystalline cellulose, microfine cellulose or a mixture thereof, a disintegrant and optionally other excipients, all percentages based on the weight of the active ingredient.

## Patentansprüche

1. Zusammensetzung, enthaltend ein Granulat, bestehend aus einem in Wasser bei Raumtemperatur in einem Verhältnis von 1:≤10 löslichen aktiven Inhaltsstoff, 0-100 Gew.-% eines Celluloseprodukts, bei dem es sich um mikrokristalline Cellulose, mikrofeine Cellulose oder einem Gemisch davon handelt, und 0-0,5 Gew.-% eines Feucht-Granulierungs-Bindemittels, wobei das Granulat hergestellt wird gemäß einem Verfahren, umfassend die Schritte:
a. Anfeuchten der das Granulat bildenden Komponente(n) mit 1-40, vorzugsweise 2,5-20 Gew.-% einer wässrigen Lösung, die gegebenenfalls das Feucht-Granulierungs-Bindemittel enthält;
b. Zerkleinern der feuchten Masse durch ein mindestens 2 mm-Sieb;
c. Trocknen des in Schritt b erhaltenen Granulats;
d. Absieben des getrockneten Granulats durch ein mindestens 0,71 mm-Sieb,
eines Celluloseprodukts, wobei es sich um mikrokristalline Cellulose, mikrofeine Cellulose oder einem Gemisch davon handelt, eines Sprengmittels und gegebenenfalls weiterer Inhaltsstoffe, wobei sich alle Prozentangaben auf das Gewicht des aktiven Inhaltsstoffes beziehen.

## Revendications

1. Composition contenant un granulé, constitué d'un ingrédient actif, soluble dans l'eau à température ambiante dans un rapport de 1:≤10, 0-100% en poids d'un produit cellulosique, qui est de la cellulose microcristalline, de la cellulose microfine ou un mélange de celles-ci, et 0-0,5% en poids d'un agent de liaison de granulation par voie humide, le granulé étant préparé selon un procédé comprenant les étapes :
a) d'humidification du ou des composant(s) pour en faire le granulé avec 1-40, de préférence 2,5-20% en poids, d'une solution aqueuse contenant facultativement l'agent de liaison de granulation par voie humide ;
b) de pulvérisation de la masse humide à travers un tamis d'au moins 2 mm ;
c) de séchage du granulé obtenu dans l'étape b ;
d) de tamisage du granulé séché à travers un tamis d'au moins 0,71 mm,
un produit de cellulose, qui est de la cellulose microcristalline, de la cellulose microfine ou un mélange de celles-ci, un désintégrant et facultativement d'autres excipients, tous les pourcentages étant donnés en poids par rapport à l'ingrédient actif.
